# EUROPÄISCHE PATENTSCHRIFT

(11) **EP 1 023 261 B1**
(45) Veröffentlichungstag und Bekanntmachung des Hinweises auf die Patenterteilung: **06.03.2002**
(21) Anmeldenummer: 98951488.0
(22) Anmeldetag: 02.10.1998
(51) Int. Cl.: C07C 209/36

(54) **KONTINUIERLICHES VERFAHREN ZUR HERSTELLUNG VON AROMATISCHEN AMINEN**
CONTINUOUS METHOD FOR PRODUCING AROMATIC AMINES
PROCEDE CONTINU DE PREPARATION D'AMINES AROMATIQUES

(30) Priorität: 15.10.1997 DE 19745465
(43) Veröffentlichungstag der Anmeldung: 02.08.2000
(73) Patentinhaber: BAYER AG, 51368 Leverkusen (DE)
(72) Erfinder: BECKHAUS, Heiko, D-51381 Leverkusen (DE); LANGER, Reinhard, D-47800 Krefeld (DE)
(86) Internationale Anmeldenummer: EP9806275
(87) Internationale Veröffentlichungsnummer: WO9919292

(56) Entgegenhaltungen:
- EP-A- 0 124 010
- EP-A- 0 634 391
- EP-A- 0 696 573
- WO-A-97/20804
- DE-A- 2 135 155

## Beschreibung

Die Erfindung betrifft ein kontinuierliches Verfahren zur Herstellung von aromatischen Di- und Polyaminen durch katalytische Hydrierung der den Aminen entsprechenden Di- und Polynitroverbindungen bei hohen Temperaturen und gegebenenfalls gleichzeitiger Wärmeabfuhr aus dem Reaktionsgemisch zur Erzeugung von Dampf mit einem Überdruck von >1,5 bar abs. an einem Festbett.

Verfahren zur Herstellung von aromatischen Aminen durch katalytische Hydrierung der ihnen zugrundeliegenden Nitroverbindungen sind in großer Zahl bekannt (DE-A-2 135 155, DE-A-2 106 644, DE-A-4 435 839, EP-A-0 696 573 und WO 97/20 804).

Allgemein wird die großtechnische, katalytische Hydrierung von aromatischen Polynitroverbindungen in Suspensionen bei niedrigen Temperaturen durchgeführt, da bei der Hydrierung von aromatischen Polynitroverbindungen bei hohen Temperaturen die Gefahr unkontrollierter Nebenreaktion besteht. Diese Nebenreaktionen können zur Bildung von unerwünschten Nebenprodukten und damit zu Ausbeuteminderungen führen. Beispielhaft erwähnt seien in diesem Zusammenhang Kernhydrierungen, hydrogenolytische Spaltungen oder die Bildung von hochmolekularen, teerartigen Produkten. Es können auch explosionsartige Nebenreaktionen ablaufen, die im stark exothermen Reaktionsverlauf der Nitrogruppenumsetzung und ihrer hohen Reaktionsgeschwindigkeit bei höheren Temperaturen begründet sind.

Bei der Umsetzung von aromatischen Polynitroverbindungen mit Wasserstoff wird eine beträchtliche Wärmemenge frei. Vorteilhaft sind die Hydrierverfahren bei denen die vermehrte Reaktionsenergie nicht mit Energieaufwand vernichtet werden muß, sondern die Reaktionsenergie wirtschaftlich in Form von Dampfgewinnung genutzt werden kann.

In den katalytischen Suspensionsverfahren sind immer wieder Verschlammungen und Ablagerungen von Katalysatoren zu beobachten. Damit sind oft Produktverunreinigungen, schlechtere Hydrierausbeuten und hohe Katalysatorverbräuche verbunden. Der Reinigungs- und Wartungsaufwand ist beträchtlich.

Diese Nachteile haben Thelen et al. (DE-A-2 135 154 und DE-A-2 135 155) durch die Beschreibung der katalytischen Hydrierung am Festbett umgangen. Trotz der vorteilhaften Anordnung des Katalysators in einem Festbett, ist allerdings nachteilig, daß die Wärmemenge im Reaktor abgeführt wird, die Dimension bzw. Konstruktion des Reaktors und die Aufbringung des spinellförmigen Katalysators auf die Kühlrohre sehr aufwendig sind. Der Durchsatz an aromatischen Polynitroverbindungen ist gering. Die Wärmeabfuhr ist problematisch und die Reaktionstemperaturen sind nur auf einem niedrigen Niveau zu führen.

In der WO 97/20 804 (Chambost et al.) wird die katalytische Hydrierung von aromatischen Nitroverbindungen am Festbett in Kombination von zwei Festbettreaktoren beschrieben. Die Aufteilung der Produktmengen nach dem ersten Reaktor mit gleichzeitiger Gasabscheidung ist problematisch und aufwendig. Nachteilig bei dem hier beschriebenen Verfahren ist die Verwendung von zwei Reaktoren und die niedrigen Reaktionstemperaturen von bis zu 120°C. Ferner werden teilweise große Mengen Lösungsmittel wie Alkohole oder Ether verwendet, wobei nach erfolgter Reaktion das Lösungsmittel vom aromataischen Diamin abgetrennt und gegebenenfalls aufbereitet werden muß.

Aufgabe war es daher, ein verbessertes Verfahren zur Herstellung von Aminen durch Hydrierung von aromatischen Nitroverbindungen zur Verfügung zu stellen, das es gestattet, auch bei hohen Temperaturen lösungsmittelfrei bzw. nur mit wenig Lösungsmittel zu arbeiten, ohne daß es zu Nebenreaktionen oder Verschlammungen kommt.

Gegenstand der Erfindung ist ein kontinuierliches Verfahren zur Herstellung von aromatischen Di- und/oder Polyaminen durch katalytische Hydrierung der entsprechenden aromatischen Di- und/oder Polynitroverbindungen mit Wasserstoff, bei dem in einem Reaktor mit katalytischem Festbett oder Rieselbett bei einem Druck im Bereich zwischen 5 und 100 bar und einer Reaktionstemperatur im Bereich von 100 bis 220°C
a) in einen Produktstrom, bestehend aus im wesentlichen rückgeführtem Hydrierprodukt, Wasser und Wasserstoff, die aromatischen Di- und/oder Polynitroverbindungen gegebenenfalls in Gegenwart eines Lösungsmittels eingebracht werden und
b) die Reaktionswärme durch einen außenliegenden Wärmetauscher abgeführt wird und
c) ein Teil des Produktstromes kontinuierlich aus dem Reaktorsystem entnommen wird.

Bevorzugt wird im Reaktor ein Druck von 10 bis 80 bar und eine Betriebstemperatur von 150 bis 200°C aufrechterhalten.

Der Produktaustrag kann an beliebiger Stelle des Reaktorsystems erfolgen. Bevorzugt erfolgt der Austrag nach dem außenliegenden Wärmetauscher vor der Umpumpung. Vorzugsweise wird der aus dem Reaktionssystem entnommene Produktstrom auf ca. 150 bis 160°C abgekühlt.

Beispiele bevorzugt eingesetzter aromatischer Nitroverbindungen sind: 1,3-Dinitrobenzol, 2,4-Dinitrotoluol, 2,6-Dinitrotoluol oder im wesentlichen aus beiden letztgenannten Isomeren bestehende, technische Dinitrotoluolgemische.

Besonders bevorzugt werden als aromatische Nitroverbindungen 2,4-Dinitrotoluol oder dessen technische Gemische mit bis zu 35 Gew.-%, bezogen auf das Gesamtgemisch, an 2,6-Dinitrotoluol eingesetzt. Diese technischen Gemische können auch untergeordnete Mengen, d.h. bis maximal 6 Gew.-%, bezogen auf das Gesamtgemisch, an 2,3-, 2,5- oder 3,4-Dinitrotoluol enthalten.

Für das erfindungsgemäße Verfahren werden die an sich bekannten Hydrierkatalysatoren für Nitroverbindungen verwandt. Die Katalysatoren können in Körperform geschüttelt, behaftet an Tragböden, Rosten, Packungen oder Geweben geometrisch wahlweise so angeordnet sein, daß der Druckverlust möglichst niedrig, die Verteilung über das Katalysatorbett optimal ist und die Reaktionsgemischgeschwindigkeit groß genug ist, um die Reaktionswärme aufzunehmen. Gut geeignete Katalysatoren sind insbesondere aus Metallen der 8. Nebengruppe des Periodensystems der Elemente, die beispielsweise auf Trägermaterialien wie Oxiden von Magnesium, Aluminium, Titan und/oder Nickel, auch Raney-Nickel, verwendet. Bevorzugt werden Nickel-Katalysatoren eingesetzt. Edelmetallkatalysatoren auf geeignetem Trägermaterial wie z.B. Palladium auf Kohle können auch Anwendung finden. Die Katalysatoren liegen bevorzugt in gepreßter Körperform, geschüttet auf Strukturböden vor.

Bevorzugt wird das erfindungsgemäße Verfahren so durchgeführt, daß
- die Umwälzung des Reaktionsgemisches über ein Festbett oder Rieselbett so betrieben wird, daß das Volumenverhältnis des Gemisches zur eingetragenen Nitroverbindung 50 bis 500, vorzugsweise 200 bis 300 beträgt,
- die Wasserstoffzufuhr selbstansaugend ist, d.h. daß sich im oberen Reaktorteil sammelnde Wasserstoffgas selbstständig durch die Energie des umgewälzten Gemisches wieder in das Reaktionsgemisch einmischt,
- der Betriebsdruck des Reaktorsystems durch Zuführung von frischem Wasserstoff von außen aufrechterhalten wird,
- das volumetrische Verhältnis von angesaugtem Wasserstoffstrom zu umgepumptem Gemisch 0,1 bis 7 beträgt, wobei der benötigte Wasserstoff aus dem Gasraum des Reaktors entzogen, und der bei der Reaktion verbrauchte Wasserstoff an beliebiger Stelle des Systems nachgeliefert wird,
- das Verhältnis Katalysator zu eingetragener Nitroverbindung <20 kg/kgh und vorzugsweise 5 bis 14 kg/kgh ist

Durch die Vermischung der aromatischen Nitrokomponente mit dem rückgeführten Hydrierprodukt (Produktloop) wird mit den übrigen Verfahrensparametern eine intensive Durchmischung und Verteilung über das Katalysatorbett erzielt. Dadurch wird eine gegebenenfalls auch lösungsmittelfreie, katalytische Hydrierung von Di- oder Polyaromaten bei hohen Temperaturen möglich, so daß durch Wärmeabfuhr aus dem System gleichzeitig Dampf von mehr als 2 bar Überdruck erzeugt werden kann. Nebenreaktionen oder dergleichen treten nicht oder nur untergeordnet auf.

Zur Regenerierung des Katalysatorbettes wird bei Bedarf die Zudosierung der aromatischen Di- und/oder Polynitroverbindungen unterbrochen. In einfacher Weise kann so durch den noch weiter fließenden Produktstrom der Katalysator regeneriert werden, ohne daß es hierfür eine längere Unterbrechung des Verfahrens bedarf.

Gegebenenfalls können die aromatischen Di- und/oder Polynitroverbindungen vorzugsweise auch in einem Lösungsmittel dem Produktstrom zudosiert werden. Als Lösungsmittel sind aliphatische C₁- bis C₄-Alkohole, insbesondere Methanol, Ethanol, Isopropanol, t-Butanol oder cyclische Ether, insbesondere Dioxan oder Tetrahydrofuran möglich.

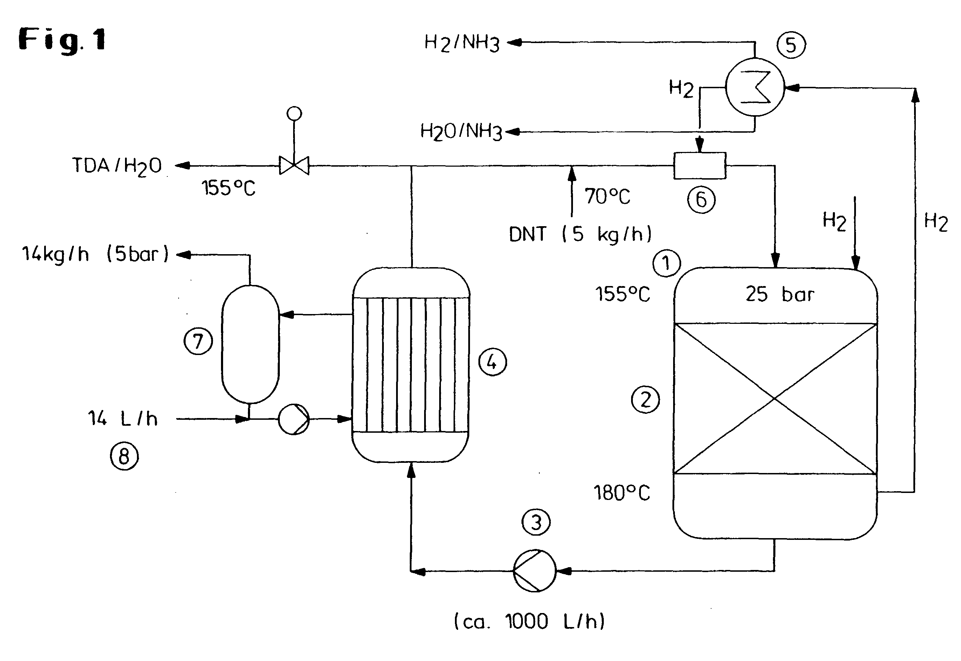

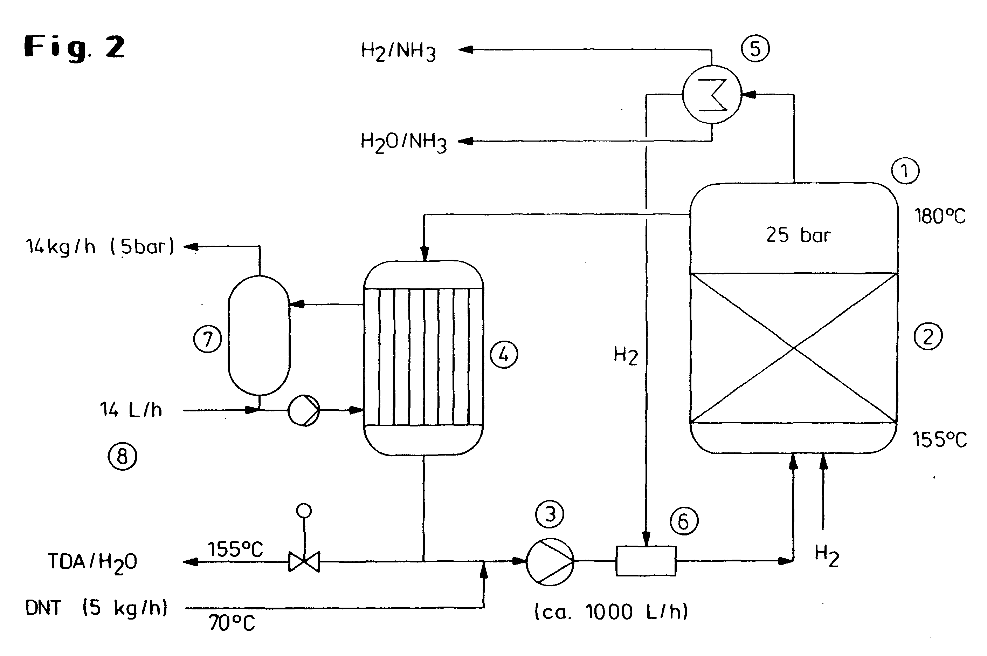

Das erfindungsgemäße Verfahren kann z.B. in einem Reaktionssystem durchgeführt werden, die in Abbildung 1 (Rieselbett) oder alternativ in Abbildung 2 (Festbett) schematisch dargestellt ist. In diesen Abbildungen haben die Zahlen folgende Bedeutung:
1) Reaktor
2) Katalysatorbett
3) Rohrsysteme, Pumpe für die Kreislaufführung des Reaktionsgemisches
4) Wärmetauscher zur Abkühlung des umgewälzten Reaktionsgemisches
5) Gaskühler
6) Ansaugung des Umlaufwasserstoffs
7) Dampfabscheider
8) Kondensat

Die Erfindung soll anhand der nachfolgenden Beispiele näher erläutert werden, ohne sie dadurch zu begrenzen.

### Beispiele

### Beispiel 1

### (vgl. Fig. 1)

In einem Autoklaven 1 (Durchmesser 14 cm) mit einem Rieselbett⁻ (Katalysator 50 l Ra-Nickel gepreßt, kubische Formkörper: 3 bis 4 mm Durchmesser, 5 bis 6 mm Höhe) werden von oben 1.000 l/h TDA-/Wassergemisch über einen Wärmetauscher 2 umgepumpt 3. Durch den von 180°C auf 155°C gekühlten Flüssigkeitsstrom wird über einen Injektor 4 die wasserstoffhaltiges, gekühltes 5 Gas aus dem Reaktor umgepumpt. Vor den Wasserstoffumlauf werden 5 kg/h Dinitrotoluol (70°C) flüssig dazugegeben. Der durch die Reaktion verbrauchte Wasserstoff wird durch frischen Wasserstoff im Gleichstrom von oben zugegeben. Entsprechend der Zudosierung von der Nitroverbindung erhält man stöchiometrisch, selektiv in >99 %iger Ausbeúte der TDA-Isomeren und Reaktionswasser.

### Beispiel 2

### (vgl. Fig. 2)

In einem Autoklaven 1 (Durchmesser 14 cm) mit einem Festbett (Katalysator 50 l wie in Beispiel 1) werden von unten 1.000 l/h TDA-/Wassergemisch über einen Wärmetauscher 2 umgepumpt. Durch den von 180°C auf 155°C gekühlten Flüssigkeitsstrom wird über einen Injektor 4 wasserstoffhaltiges, gekühltes 5 Gas aus dem Reaktor umgepumpt. Vor die Umpumpung 3 werden 5 kg/h Dinitrotoluol (70°C) flüssig zudosiert. Der durch die Reaktion verbrauchte Wasserstoff wird durch frischen Wasserstoff im Gleichstrom von unten zugegeben. Entsprechend der Zudosierung von der Nitroverbindung erhält man stöchiometrisch, selektiv in >99,2 %iger Ausbeute der TDA-Isomeren und Reaktionswasser.

## Patentansprüche

1. Kontinuierliches Verfahren zur Herstellung von aromatischen Di- und/oder Polyaminen durch katalytische Hydrierung der entsprechenden aromatischen Di- und/oder Polynitroverbindungen mit Wasserstoff, **dadurch gekennzeichnet, daß** in einem Reaktor mit katalytischem Festbett oder Rieselbett bei einem Druck im Bereich zwischen 5 und 100 bar und einer Reaktionstemperatur im Bereich von 100 bis 220°C
a) in einen Produktstrom, bestehend aus im wesentlichen rückgeführtem Hydrierprodukt, Wasser und Wasserstoff, die aromatischen Di- und/oder Polynitroverbindungen eingebracht werden und
b) die Reaktionswärme durch einen außenliegenden Wärmetauscher abgeführt wird und
c) ein Teil des Produktstromes kontinuierlich aus dem Reaktorsystem entnommen wird.

2. Verfahren gemäß Anspruch 1, bei dem die aromatischen Di- und/oder Polynitroverbindungen in Gegenward eines Lösungsmittels in dem Produktstrom eingebracht werden.

3. Verfahren gemäß Anspruch 1 bis 2, **dadurch gekennzeichnet, daß** die aromatische Di- oder Polynitroverbindung in reiner Form, als Mischung mit dem entsprechenden Di- oder Polyamin und Wasser eingesetzt wird.

4. Verfahren gemäß Anspruch 1 bis 3, **dadurch gekennzeichnet, daß** als aromatische Dinitroverbindung 2,4-Dinitrotoluol oder dessen technische Gemische mit 2,6-Dinitrotoluol eingesetzt wird.

5. Verfahren gemäß Anspruch 1 bis 4, **dadurch gekennzeichnet, daß** das volumetrische Verhältnis des umgepumpten, schnellfließenden Gemisches zur eingetragenen Di- oder Polynitroverbindung 50 bis 500 beträgt.

6. Verfahren gemäß Anspruch 1 bis 5, **dadurch gekennzeichnet, daß** das Verhältnis Katalysator zu eingetragener Nitroverbindung <20 kg/kgh, vorzugsweise 5 bis 14 kg/kgh ist.

7. Verfahren gemäß Anspruch 1 bis 6, **dadurch gekennzeichnet, daß** das Katalysatorbett entweder von oben oder von unten mit dem rückgeführten Hydrierprodukt beaufschlagt wird.

8. Verfahren gemäß Anspruch 2 bis 7, **dadurch gekennzeichnet, daß** als Lösungsmittel aliphatische C₁- bis C₄-Alkohole oder cyclische Ether eingesetzt werden.

9. Verfahren gemäß Anspruch 8, **dadurch gekennzeichnet, daß** das Lösungsmittel in einer Menge von 0,1 bis 40 Gew.-% des Reaktionsgemisches eingesetzt wird.

10. Verfahren gemäß Anspruch 8 bis 9, **dadurch gekennzeichnet, daß** das Lösungsmittel in einer Menge von 1 bis 10 Gew.-% des Reaktionsgemisches eingesetzt wird.

## Claims

1. Continuous process for the preparation of aromatic di- and/or polyamines by catalytic hydrogenation of the corresponding aromatic di- and/or polynitro compounds with hydrogen, **characterised in that**, in a reactor with a catalytic fixed bed or trickle bed at a pressure between 5 and 100 bar and a reaction temperature from 100 to 220°C,
a) the aromatic di- and/or polynitro compounds are introduced into a product stream comprising recycled hydrogenated product, water and hydrogen, and
b) the reaction heat is carried off by an external heat exchanger, and
c) a part of the product stream is removed continuously from the reactor system..

2. Process according to claim 1, in which the aromatic di- and/or polynitro compounds are introduced in the presence of a solvent.

3. Process according to claims 1 to 2, **characterised in that** the aromatic di- or polynitro compound in the pure form is used as a mixture with the corresponding di- or polyamine and water.

4. Process according to claims 1 to 3, **characterised in that** the aromatic dinitro compound used is 2,4-dinitrotoluene or industrial mixtures thereof with 2,6-dinitrotoluene.

5. A process according to claims 1 to 4, **characterised in that** the volumetric ratio of the pumped, rapidly flowing mixture to the di- or polynitro compound introduced is 50 to 500.

6. Process according to claims 1 to 5, **characterised in that** the ratio of catalyst to nitro compound introduced is 20 kg/kgh, preferably 5 to 14 kg/kgh.

7. Process according to claims 1 to 6, **characterised in that** the catalyst bed is impinged either from above or from below with the recycled hydrogenated product.

8. Process according to claim 2 to 7, **characterised in that** solvents used are aliphatic C₁ to C₄ alcohols or cyclic ethers.

9. Process according to claim 8, **characterised in that** the solvent is used in a quantity of 0.1 to 40 wt.% of the reaction mixture.

10. Process according to claims 9 to 10, **characterised in that** the solvent is used in a quantity of 1 to 10 wt.% of the reaction mixture.

## Revendications

1. Procédé continu pour la préparation de di- et/ou polyamines par hydrogénation catalytique des composés aromatiques di- et/ou polynitrés correspondants à l'aide d'hydrogène, **caractérisé en ce qu'**on introduit dans un réacteur à lit catalytique fixe ou fluidifié, à une pression comprise dans la plage s'étendant entre 5 et 100 bar et à une température de réaction comprise dans la plage allant de 100 à 220°C,
a) les composés aromatiques di- et/ou polynitrés dans un courant de produit constitué de produit d'hydrogénation recyclé pour l'essentiel, d'eau et d'hydrogène et
b) on prélève la chaleur de réaction par un échangeur de chaleur situé à l'extérieur et
c) on soutire du système de réacteur une partie du courant de produit, en continu.

2. Procédé suivant la revendication 1, dans lequel les composés di- et/ou polynitrés sont introduits dans le courant de produit en présence d'un solvant.

3. Procédé suivant l'une des revendications 1 à 2, **caractérisé en ce qu'**on met en oeuvre les composés aromatiques di- ou polynitrés sous forme pure, en mélange avec les di- ou polyamines correspondantes et l'eau.

4. Procédé suivant l'une des revendications 1 à 3, **caractérisé en ce qu'**on met en oeuvre comme composé dinitré le 2,4-dinitrotoluène ou un mélange technique de celui-ci avec le 2,6-dinitrotoluène.

5. Procédé suivant l'une des revendications 1 à 4, **caractérisé en ce que** le rapport volumétrique entre le mélange transvasé par pompage, en écoulement rapide, et le composé di- ou polynitré a une valeur de 50 à 500.

6. Procédé suivant l'une des revendications 1 à 5, **caractérisé en ce que** le rapport entre le catalyseur et le composé nitré introduit a une valeur inférieure à 20 kg/kgh, de préférence une valeur de 5 à 14 kg/kgh.

7. Procédé suivant l'une des revendications 1 à 6, **caractérisé en ce que** le lit catalytique est mis en contact avec le produit d'hydrogénation recyclé soit à partir du haut, soit à partir du bas.

8. Procédé suivant l'une des revendications 2 à 7, **caractérisé en ce qu'**on met en oeuvre comme solvant des alcools aliphatiques en C₁ à C₄ ou des éthers cycliques.

9. Procédé suivant la revendications 8, **caractérisé en ce qu'**on met en oeuvre le solvant en une quantité de 0,1 à 40% en poids par rapport au mélange réactionnel.

10. Procédé suivant l'une des revendications 8 à 9, **caractérisé en ce qu'**on met en oeuvre le solvant en une quantité de 1 à 10% en poids par rapport au mélange réactionnel.
